(19) Europäisches Patentamt
European Patent Office
Office européen des brevets

(11) Publication number: 0 046 337 A2


(12) # EUROPEAN PATENT APPLICATION


(21) Application number: 81303269.5

(22) Date of filing: 16.07.81

(51) Int. Cl.[3]: **C 07 D 409/06**, C 07 D 405/06, C 07 D 403/06, C 07 D 401/06, C 07 D 409/04, C 07 D 407/04, C 07 D 405/04, C 07 D 333/16, C 07 D 333/22, C 07 D 307/42, C 07 D 307/46

(30) Priority: 20.08.80 GB 8027070
08.12.80 GB 8039279
29.01.81 GB 8102704
23.03.81 GB 8109025

(43) Date of publication of application: 24.02.82 Bulletin 82/8

(84) Designated Contracting States: AT BE CH DE FR GB IT LI LU NL SE



(71) Applicant: IMPERIAL CHEMICAL INDUSTRIES PLC
Imperial Chemical House Millbank, London SW1P 3JF (GB)

(72) Inventor: Freeman, Peter Hilary, 7 Silverdale Road, Earley Berkshire (GB)
Inventor: Worthington, Paul Anthony, 4 Oakhurst Road Maidenhead Court Park, Maidenhead Berkshire (GB)
Inventor: Rathmell, William George, Culvers 10 Gypsy Lane, Wokingham Berkshire (GB)

(74) Representative: Alner, Henry Giveen Hamilton et al, Imperial Chemical Industries PLC Legal Department: Patents Thames House North Millbank, London SWIP 4QG (GB)


(54) **Triazole compounds, a process for preparing them, their use as plant and pharmaceutical fungicides and as plant growth regulators and compositions containing them.**


(57) Racemic compounds of formula:

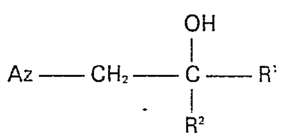

and stereoisomers thereof, wherein $R^1$ is alkyl, cyclo alkyl or optionally substituted phenyl; $R^2$ is a heterocyclic group; and Az is 1,2,4-triazolyl radical; and their acid addition salts, ethers, esters and metal complexes; have fungicidal activity useful in the agricultural and pharmaceutical fields. They are also capable of regulating plant growth.



EP 0 046 337 A2

# TRIAZOLE COMPOUNDS, A PROCESS FOR PREPARING THEM, THEIR USE AS PLANT AND PHARMACEUTICAL FUNGICIDES AND AS PLANT GROWTH REGULATORS AND COMPOSITIONS CONTAINING THEM

This invention relates to triazole and imidazole compounds useful as fungicides, to a process for preparing them, to fungicidal and plant growth regulating compositions containing them, to processes using them to combat fungal infections in plants, and to regulate plant growth. The invention also relates to pharmaceutical and veterinary compositions comprising triazole compounds, and in particular to such compositions which are orally or topically suitable for administration to humans or other animals for the treatment of fungus diseases. These compositions of the invention are especially useful for treatment of candidiasis and human dermatophyte infections.

The invention provides a racemic compound having the formula (I):

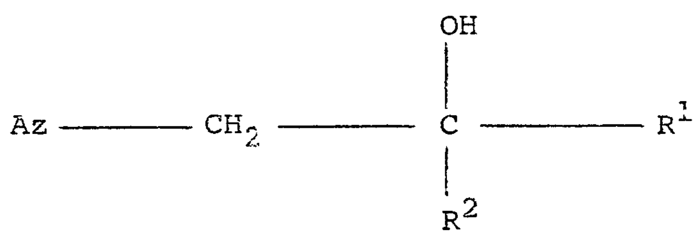

formula (I)

or a stereoisomer thereof, wherein $R^1$ is alkyl, cycloalkyl (e.g. cyclopropyl, cyclopentyl or cyclohexyl) or optionally substituted phenyl; $R^2$ is a heterocyclic group; and Az is a 1, 2, 4-triazolyl radical; and acid addition salts, ethers, esters and metal complexes thereof. Az represents a 1,2, 4-triazol-1-yl radical or a 1, 2, 4-triazol-4-yl radical.

The compounds of the invention are generally obtained in the form of racemic mixtures which can be separated into the individual isomers by methods known in the art.

BAD ORIGINAL

The alkyl groups can be a straight or branched chain group having 1 to 6, e.g. 1 to 4, carbon atoms; examples are methyl, ethyl, propyl (n- or iso-propyl) and butyl (n-, sec-, iso- or t-butyl).

Examples of suitable substituents for the phenyl are halogen (e.g. fluorine, chlorine or bromine), $C_{1-5}$ alkyl [e.g. methyl, ethyl, propyl (n- or iso-propyl) and butyl (n-, sec-, iso- or t-butyl)], $C_{1-4}$ alkoxy (e.g. methoxy, and ethoxy), halo-alkoxy (e.g.trifluoromethoxy), trifluoromethyl, nitro, phenyl and phenoxy.

Suitably the phenyl is unsubstituted or substituted with 1 2 or 3 ring substituents as defined above. Preferably the phenyl has a single ring substituent in the 2-position. Examples of these groups are phenyl, 2-, 3- or 4-chlorophenyl, 2,4- or 2,6-dichlorophenyl, 2-, 3- or 4-fluorophenyl, 2,6-difluorophenyl, 2-, 3- or 4-bromophenyl, 2-chloro-4-fluorophenyl, 2-chloro-6-fluorophenyl, 2-, 3- or 4-methoxyphenyl, 2,4-dimethoxyphenyl, 2-, 3- or 4-ethoxyphenyl, 2-, 3- or 4-nitrophenyl, 2-, 3- or 4-methylphenyl, 2-, 3- or 4-t-butylphenyl, 2-, 3- or 4-trifluoromethylphenyl, 2-, 3- or 4-phenoxyphenyl, and 2-, 3- or 4-phenylphenyl (2-, 3- or 4-biphenylyl).

The heterocyclic group, which may be unsubstituted or substituted may be, for example a thiophene, furan, pyridine, pyrimidine or pyrrole ring. Substituents on the ring may be, for example, halogen (especially chlorine) or alkyl, (especially $C_{1-4}$ alkyl, and particularly t-butyl). Especially preferred compounds bear the groups

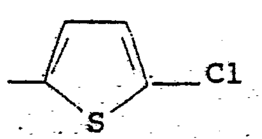

or

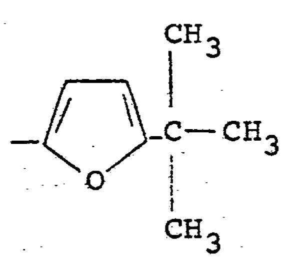

BAD ORIGINAL

# 0046337



In a further aspect therefor, the invention provides a triazole compound as defined above wherein $R^1$ is an alkyl group which is a straight or branched chain alkyl group having from 1 to 6, especially 1 to 4 , carbon atoms.

In another aspect the invention provides a triazole compound as defined above wherein $R^1$ is phenyl or phenyl substituted with halogen, $C_{1-5}$ alkyl, $C_{1-4}$ alkoxy, halo-alkoxy, trifluoromethyl, nitro, phenyl or phenoxy.

In yet another aspect the invention provides a triazole compound as defined above wherein $R^1$ is phenyl or phenyl substituted with 1, 2 or 3 ring substituents.

In a still further aspect the invention provides a triazole compound as defined above wherein $R^1$ is phenyl 2-, 3-, or 4-chlorophenyl, 2,4- or 2,6-dichlorophenyl, 2-, 3-, or 4-fluorophenyl, 2,6-difluorophenyl, 2-, 3-, or 4-bromophenyl, 2-chloro-4-fluorophenyl, 2-chloro-6-fluorophenyl, 2-, 3-, or 4-methoxyphenyl, 2, 4-dimethoxyphenyl, 2-, 3-, or 4-ethoxyphenyl, 2-, 3-, or 4-nitrophenyl, 2-, 3-, or 4-methylphenyl, 2-, 3- or 4-_t_-butylphenyl, 2-, 3-, or 4-trifluoromethylphenyl, 2-, 3-, or 4-phenoxyphenyl, and 2-, 3-, or 4-phenylphenyl (2-, 3-, or 4-biphenylyl).

In a yet further aspect the invention provides a triazole compound as defined above wherein $R^2$ is an unsubstituted, or halo- or alkyl-substituted, thiophene, furan, pyridine, pyrimidine or pyrrole ring. Preferably $R^2$ is thienyl and a particularly preferred value for $R^2$ is 2-(5-chlorothienyl) more especially when $R^1$ is phenyl or halophenyl.

In a preferred aspect the invention provides a compound having the formula :

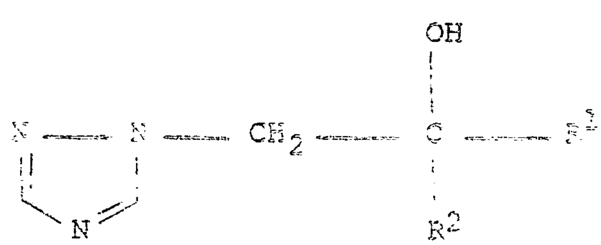

BAD ORIGINAL

wherein $R^1$ is phenyl, 2- or 4-, chloro- or fluoro-phenyl, or 2, 4-dichlorophenyl, or 2, 4-difluorophenyl, and $R^2$ is 2-thienyl, 2-[5-chlorothienyl], 2-furyl, 2-(5-butylfuryl), 2-, 3- or 4-pyridyl or 2-thienyl. $R^2$ is preferably 2-thienyl or 2-[5-chlorothienyl] when $R^1$ is phenyl or halophenyl.

A particularly preferred compound provided by the invention is :

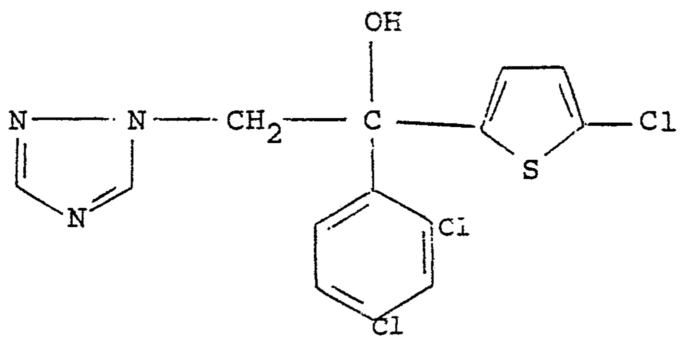

The salts can be salts with inorganic or organic acids e.g. hydrochloric, nitric, sulphuric, acetic, 4-toluenesulphonic or oxalic acid.

Suitably the metal complex is one including, as the metal, copper, zinc, manganese or iron. It preferably has the general formula:

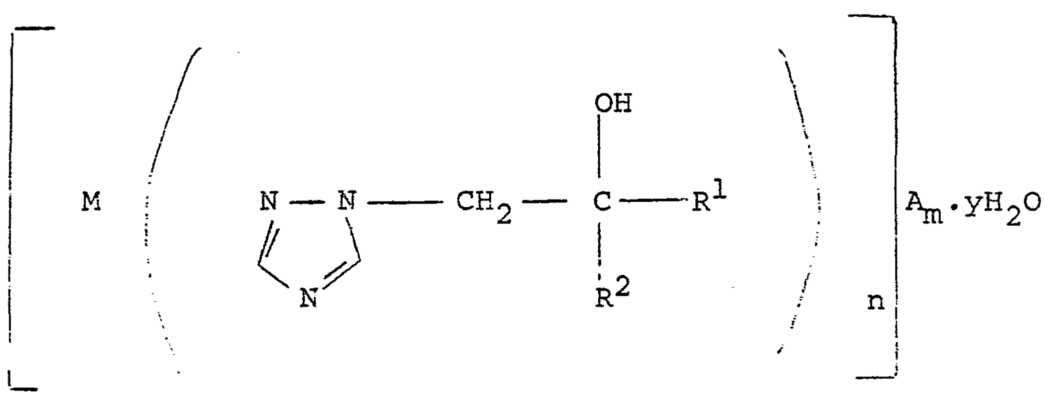

wherein $R^1$ and $R^2$ are as defined above, M is a metal, A is an anion (e.g. a chloride, bromide, iodide, nitrate, sulphate or phosphate anion), n is 2 or 4, y is 0 or an integer of 1 to 12, and m is an integer consistent with valency.

Examples of the compounds of the invention corresponding to the general formula :

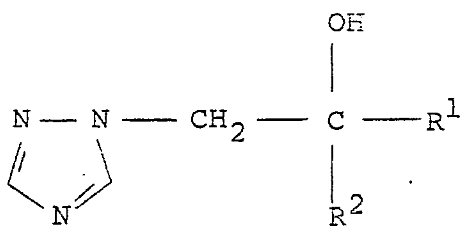

Structure A

and

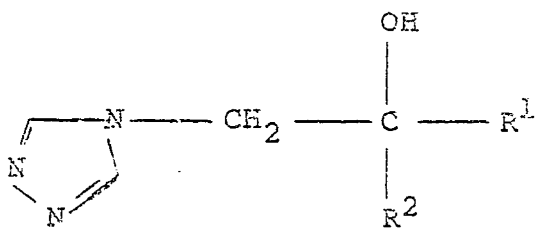

Structure B

are shown in Table I below and the physical characteristics (melting points) are shown in columns A and B for the appropriate structure.

TABLE I

| COMPOUND NO | | $R^1$ | $R^2$ | MELTING POINTS °C | |
|---|---|---|---|---|---|
| A | B | | | Structure A | Structure B |
| 1A | 1B | phenyl | 2-thienyl | 103-104 | 224-225 |
| 2A | 2B | 4-chlorophenyl | " | | 203-204 |
| 3A | 3B | 2,4-dichlorophenyl | " | 140-141 | 220-222 |
| 4A | | 2-chlorophenyl | " | | |
| 5A | | 2-fluorophenyl | " | | |
| 6A | 6B | 4-fluorophenyl | " | 98-99 | 198-199 |
| 7A | | 2-methylphenyl | " | | |
| 8A | 8B | 4-methylphenyl | " | 110-111 | 208-209 |
| 9A | 9B | phenyl | 2-[5-chlorothienyl] | 165-166 | 257-258 |
| 10A | 10B | 4-chlorophenyl | " | 148-149 | 248-250 |
| 11A | 11B | 2,4-dichlorophenyl | " | 139-140 | 247-248 |
| 12A | | 2-chlorophenyl | " | | |
| 13A | | 2-fluorophenyl | " | | |
| 14A | | 4-fluorophenyl | " | | |
| 15A | | 2-methylphenyl | " | | |
| 16A | | 4-methylphenyl | " | | |
| 17A | | phenyl | 2-furyl | | |
| 18A | | 4-chlorophenyl | " | 120-121 | |
| 19A | | 2,4-dichlorophenyl | " | 149-150 | |
| 20A | | 2-chlorophenyl | " | | |

TABLE I

| COMPOUND NO | | $R^1$ | $R^2$ | MELTING POINTS °C | |
|---|---|---|---|---|---|
| A | B | | | Structure A | Structure B |
| 20A | | 2-chlorophenyl | " | | |
| 21A | 21B | 2-fluorophenyl | " | | 188-189 |
| 22A | 22B | 2,4-difluorophenyl | " | | 211-212 |
| 23A | | 2-methylphenyl | " | | |
| 24A | | 4-methylphenyl | " | | |
| 25A | | phenyl | 2-[5-butylfuryl] | | |
| 26A | | 4-chlorophenyl | 2-[5-butylfuryl] | | |
| 27A | | 2,6-dichlorophenyl | " | | |
| 28A | | 2-chlorophenyl | " | | |
| 29A | | 2-fluorophenyl | 2-[5-butylfuryl] | | |
| 30A | | 4-fluorophenyl | " | | |
| 31A | | 4-methylphenyl | " | | |
| 32A | | 2-methylphenyl | " | | |
| 33A | 33B | phenyl | 2-pyridyl | 98-99 | 206-207 |
| 34A | | 4-chlorophenyl | " | | |
| 35A | | 2,4-dichlorophenyl | " | | |
| 36A | | 2-chlorophenyl | " | | |
| 37A | | 2-fluorophenyl | " | | |
| 38A | | 4-fluorophenyl | " | | |
| 39A | | 2-methylphenyl | " | | |
| 40A | | 4-methylphenyl | " | | |

TABLE I

| COMPOUND NO | | $R^1$ | $R^2$ | MELTING POINTS °C | |
|---|---|---|---|---|---|
| A | B | | | Structure A | Structure B |
| 41A | | 4-trifluoromethyl | 2-thienyl | | |
| 42A | | chlorophenyl | " | | |
| 43A | | 2,4-dimethylphenyl | " | | |
| 44A | | 4-phenoxyphenyl | " | | |
| 45A | | 2-methoxyphenyl | " | | |
| 46A | | 2-ethoxyphenyl | " | | |
| 47A | | 2,4-dimethoxyphenyl | " | | |
| 48A | | 2-nitrophenyl | " | | |
| 49A | | 4-t-butylphenyl | " | | |
| 50A | | 4-phenylphenyl | " | | |
| 51A | | phenyl | 4-pyridyl | 177-178 | |
| 52A | | 4-chlorophenyl | " | 186-187 | |
| 53A | | 2,4-dichlorophenyl | " | | |
| 54A | | 2-chlorophenyl | " | | |
| 55A | | 2-fluorophenyl | " | | |
| 56A | | 4-fluorophenyl | " | | |
| 57A | | 2-methylphenyl | " | | |
| 58A | | 4-methylphenyl | " | | |
| 59A | | 4-methoxyphenyl | " | | |
| 60A | | 2,4-difluorophenyl | 2-thienyl | 101-103 | |

The compounds of general formula (I) may be produced by reacting a compound of general formula (II) or (III):

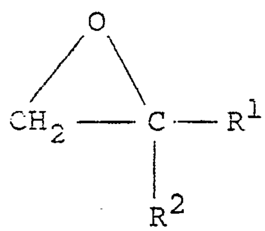

(II)

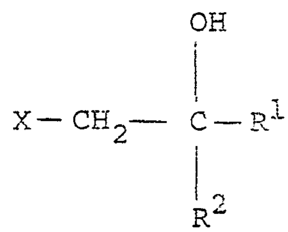

(III)

in which $R^1$ and $R^2$ are as defined above and X is a halogen atom (preferably a chlorine or bromine atom), with 1,2,4-triazole either in the presence of an acid-binding agent or in the form of one of its alkali metal salts in a convenient solvent. Suitably the compound of general formula (II) or (III) is reacted at 20-100°C with the sodium salt of 1,2,4-triazole (the salt can be prepared by adding either sodium hydride or sodium methoxide to 1,2,4-triazole) in a convenient solvent such as acetonitrile, methanol, ethanol or dimethylformamide. The product can be isolated by pouring the reaction mixture into water and recrystallising the solid formed from a convenient solvent.

The compounds of general formula (II) and (III) can be prepared by reacting a compound of general formula (IVa) or (IVb):

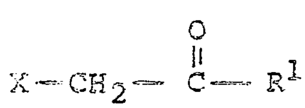

(IVa)

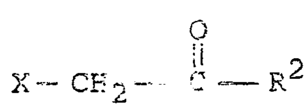

(IVb)

wherein $R^1$, $R^2$ and X are as defined above with, respectively, a Grignard compound of general formula (Va) or (Vb):

$$Y-Mg-R^2 \qquad Y-Mg-R^1$$

(Va) (Vb)

wherein $R^1$ and $R^2$ are as defined above and Y is a halogen (preferably chlorine, bromine or iodine) in a convenient solvent such as diethyl ether or tetrahydrofuran. Generally a mixture of the compounds of general formula (II) and (III) are obtained. For example, when a compound of general formula (IVa) wherein $R^1$ is alkyl or cycloalkyl is reacted, the compound of formula (II) generally predominates in the mixture; on the other hand, when $R^1$ is optionally substituted phenyl, the compound of general formula (III) generally predominates in the mixture.

The compounds of formula (II) and (III) are valuable intermediates and as such form part of the invention.

The compounds of general formula (IV) and (V) may be made by methods set out in the literature.

The compounds of general formula (II) wherein $R^1$ is unsubstituted or substituted phenyl and $R^2$ is a heterocyclic group may also be prepared by reacting the appropriate ketone of general formula (VI)

$$R^1-CO-R^2$$

(VI)

wherein $R^1$ and $R^2$ are as defined above, with dimethyl oxosulphonium methylide (Corey and Chaykovsky, JACS, 1965, 87, 1353-1364) or dimethyl sulphonium methylide (Corey and Chaykovsky, JACS, 1962, 84, 3782) using methods set out in the literature.

The ketones of general formula (VI) can be prepared, using the the Friedel-Crafts reaction, or by methods set out in the literature. They are valuable intermediates and form part of the invention.

The compounds of general formula (II) wherein $R^1$ is alkyl, cycloalkyl or optionally substituted phenyl and $R^2$ is a heterocyclic group can also be produced by reacting a β-hydroxy selenide compound of general formula (VII)

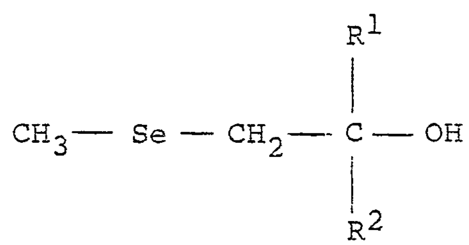

(VII)

wherein $R^1$ and $R^2$ are as defined above, with methyl iodide in potassium *t*-butoxide according to the method of Van Ende, Dumont and Krief, Angew. Chem. Int. Ed., 1975, 14, 700.

The β-hydroxy selenide compound can be prepared by treating the diselenide with the appropriate ketone in the presence of butyl lithium.

The salts and metal complexes of the compounds of general formula (I) can be prepared from the latter in known manner. For example, the complexes can be made by reacting the uncomplexed compound with a metal salt in a suitable solvent.

The ethers are made by treating the sodium salt of the alcohol with a reactive halogenated compound (e.g. methyl bromide or iodide, benzyl chloride, or allyl bromide). The esters are made in a similar manner by treating the sodium salt of the alcohol with an acid chloride (e.g. acetyl chloride, benzoyl chloride or methane sulphonyl chloride).

The compounds and salts, metal complexes, ethers and esters thereof, are active fungicides, particularly against the diseases:-

Piricularia oryzae on rice
Puccinia recondita, Puccinia striiformis and other rusts on wheat, Puccinia hordei, Puccinia striiformis and other rusts on barley, and rusts on other hosts e.g. coffee, apples, vegetables and ornamental plants
Erysiphe graminis (powdery mildew) on barley and wheat and other powdery mildews on various hosts such as Sphaerotheca fuliginea on cucurbits (e.g. cucumber), Podosphaera leucotricha on apples and Uncinula necator on vines
Helminthosporium spp. and Rhynchosporium spp. on cereals
Cercospora arachidicola on peanuts and other Cercospora species on for example suggar beet, bananas and soya beans
Botrytis cinerea (grey mould) on tomatoes, strawberries, vines and other hosts
Venturia inaequalis (scab) on apples

Some of the compounds have also shown a broad range of activities against fungi in vitro. They have activity

against various post-harvest diseases on fruit (e.g. Penicillium digatatum and italicum on oranges and Gloeosporium musarum on bananas). Further some of the compounds are active as seed dressings against: Fusarium spp., Septoria spp., Tilletia spp. (i.e. bunt, a seed borne disease of wheat), Ustilago spp., Helminthosporium spp. on cereals, Rhizoctonia solani on cotton and Corticium sasakii on rice.

The compounds can move acropetally in the plant tissue. Moreover, the compounds can be volatile enough to be active in the vapour phase against fungi on the plant.

The compounds, and their derivatives as defined above, also have plant growth regulating activities.

The plant growth regulating effects of the compounds are manifested as for example a stunting or dwarfing effect on the vegetative growth of woody and herbaceous mono- and di-cotyledonous plants. Such stunting or dwarfing may be useful, for example, in peanuts, cereals and soya bean where reduction in stem growth may reduce the risk of lodging and may also permit increased amounts of fertiliser to be applied. The stunting of woody species is useful in controlling the growth of undergrowth under power lines etc. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. Examples of suitable grasses are Stenotaphrum secundatum (St. Augustine grass), Cynosurus cristatus, Lolium multiflorum and perenne, Agrostis tenuis, Cynodon dactylon (Bermuda grass), Dactylis glomerata, Festuca spp. (e.g. Festuca rubra) and Poa spp. (e.g. Poa pratense). The compounds may stunt grasses without significant phytotoxic effects and without

BAD ORIGINAL

deleteriously affecting the appearance (particularly the colour) of the grass; this makes such compounds attractive for use on ornamental lawns and on grass verges. They may also have an effect on flower head emergence in for example grasses. The compounds can also stunt weed species present in the grasses; examples of such weed species are sedges (e.g. Cyperus spp.) and dicotyledonous weeds (e.g. daisy, plantain, knotweed, speedwell, thistle, docks and ragwort). The growth of non-crop vegetation (e.g. weeds or cover vegetation) can be retarded thus assisting in the maintenance of plantation and field crops. In fruit orchards, particularly orchards subject to soil erosion, the presence of grass cover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species may be stunted more than another grass species; this selectivity could be useful for example for improving the quality of a sward by preferential suppression of the growth of undesirable species.

The dwarfing may also be useful in miniaturising ornamental, household, garden and nursery plants (e.g. poinsettias, chrysanthemums, carnations, tulips and daffodils).

As indicated above, the compounds can also be used to stunt woody species. This property can be used to control hedgerows or to shape fruit trees (e.g. apples). Some coniferous trees are not significantly stunted by the compounds so the compounds could be useful in controlling undesirable vegetation in conifer nurseries.

The plant growth regulating effect may (as implied above) manifest itself in an increase in crop yield.

In the potato, vine control in the field and inhibition of sprouting in the store may be possible.

Other plant growth regulating effects caused by the compounds include alteration of leaf angle and promotion of tillering in monocotyledonous plants. The former effect may be useful for example in altering the leaf orientation of, for example, potato crops thereby letting more light into the crops and inducing an increase in phytosynthesis and tuber weight. By increasing tillering in monocotyledonous crops (e.g. rice), the number of flowering shoots per unit area may be increased thereby increasing the overall grain yield of such crops. In grass swards an increase in tillering could lead to a denser sward which may result in increased resilience in wear.

The treatment of plants with the compounds can lead to the leaves developing a darker green colour.

The compounds may inhibit, or at least delay, the flowering of sugar beet and thereby may increase sugar yield. They may also reduce the size of sugar beet without reducing significantly the sugar yield thereby enabling an increase in planting density to be made. Similarly in other root crops (e.g. turnip, swede, mangold, parsnip, beetroot, yam and cassava) it may be possible to increase the planting density.

The compounds could be useful in restricting the vegetative growth of cotton thereby leading to an increase in cotton yield.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates the compounds can have a growth stimulating effect on plants.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.1 to 15, preferably 0.1 to 5, kg per hectare is used. However, on certain plants even application rates within these ranges may give undesired phytotoxic effects. Routine tests may be necessary to determine the best rate of application of a specific compound for any specific purpose for which it is suitable.

The compounds may be used as such for fungicidal or plant growth regulating purposes but are more conveniently formulated into compositions for such usage. The invention thus further provides a fungicidal or plant growth regulating composition comprising a compound of general formula (I) as hereinbefore defined, or a salt, metal complex, ether or ester thereof; and, optionally, a carrier or diluent.

The invention also provides a method of combating fungi, which comprises applying to a plant, to seed of a plant or to the locus of a plant or seed, a compound, or salt, metal complex, ether or ester thereof, as hereinbefore defined.

The invention also provides a method of regulating plant growth which comprises applying to a plant, to seed of a plant or to the locus of a plant or seed, a compound, or salt, metal complex, ether or ester thereof, as hereinbefore defined.

The compounds, salts, metal complexes, ethers and esters can be applied in a number of ways, for example they can be formulated or unformulated, directly to the

foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a

wetting agent to facilitate the disperion in liquids of the powder or grains which may contain also fillers and puspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating, for example coated with, the compound, are preferred. Such granules suitably contain up to 25% by weight of the compound. The invention therefore also provides a fertiliser composition comprising the compound of general formula (I) or a salt, metal complex, ether or ester complex thereof.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-anionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after such storage be capable of dilution with water in order to form aqueous preparations which remain homogenous for a sufficient time to enable them to be applied by conventional

spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylene-sulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal or plant growth regulating activity or compounds having herbicidal or insecticidal activity.

The other fungicidal compound can be for example one which is capable of combating ear diseases of cereals (e.g. wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, dimethirimol, bupirimate, chlorothalonil, vinclozolin,

procymidone, iprodione, metalaxyl, fosetyl-aluminium, carboxin, oxycarboxin, fenarimol, nuarimol, fenfuram, methfuroxam, nitrotal-isopropyl, triadimefon, thiabendazole, etridiazole, triadimenol, biloxazol, dithianon, binapacryl, quinomethionate, guazatine, dodine, fentin acetate, fentin hydroxide, dinocap, folpet, dichlofluanid, ditalimphos, kitazin, fenpropemorph, cycloheximide, dichlobutrazol, a dithiocarbamate, a copper compound, a mercury compound, DPX 3217, RE 216[illegible], Chevron RE 20615, CGA 64250, CGA 54251 and RO 14-3169.

Suitable insecticides are pirimor, croneton, dimethoate, metasystox and formothion.

The other plant growth regulating compound can be one which controls weeds or seedhead formation, improves the level or longevity of the plant growth regulating activity of the compounds of general formula (I), selectively controls the growth of the less desirable plants (e.g. grasses) or causes the compound of general formula (I) to act faster or slower as a plant growth regulating agent. Some of these other agents will be herbicides. Examples of suitable agents are the gibberellins (e.g. $GA_3$, $GA_4$ or $GA_7$), the auxins (e.g. indoleacetic acid, indolebutyric acid, naphthoxyacetic acid or naphthylacetic acid), the cytokinins (e.g. kinetin, diphenylurea, benzimidazole, benzyladenine or BAP), phenoxyacetic acids (e.g. 2,4-D or MCPA), substituted benzoic acids (e.g. TIBA), morphactins (e.g. chlorfluorecol), maleic hydrazide, glyphosate, glyphosine, long chain fatty alcohols and acids (e.g. Off Shoot O or Off Shoot T), dikegulac, Sustar, Embark, substituted quaternary ammonium and phosphonium compounds (e.g. CCC or Phosfon-D), Ethrel, carbetamide, Racuza, Alar, asulam, abscissic acid, isopyrimol, RH531, hydroxybenzonitriles (e.g. bromoxynil), Avenge, Suffix or Lontrel.

BAD ORIGINAL

As indicated above the compounds of the invention possess antifungal properties which are useful in the treatment of candidiasis and human dermatophyte infections. For this purpose they are conveniently formulated into pharmaceutical and veterinary compositions.

The pharmaceutical and veterinary compositions of the invention may be in a conventional pharmaceutical form suitable for oral administration, for example a tablet, a capsule, an emulsion or an aqueous or oily solution or suspension, or suitable for topical application, for example a cream, ointment or gel. The composition may contain conventional pharmaceutical excipients, and may be manufactured by conventional pharmaceutical techniques.

Preferred pharmaceutical or veterinary compositions of the invention are compositions suitable for oral administration, and particularly tablets and capsules.

The antifungal activity of the active ingredients of the compositions of the invention against Candida albicans, a causative fungus of candidiasis, and Trichophyton mentagrophytes, var. quinkeanum, was demonstrated as follows:-

Female mice of around 30 g weight are injected subcutaneously on a Friday with 0.5 mg of oestradiol benzoate. The following Monday (day 0) they are clipped on the back and then dosed orally with test compounds. They are then inoculated with Candida albicans in the vagina and Trichophyton mentagrophytes var. quinkeanum on the back, and then given a second dose of the same compound. Dosing is repeated once daily on days 1 to 4. On day 7 skin lesions are scored visually and vaginal samples taken for culture on agar. Groups of 5 mice are used and compounds are dosed initially at a level of 250 mg/kg. The dose is then reduced sequentially until a minimum effective dose (MED) is found, which was 5 mg/kg against Candida and 50 mg/kg against Trichophyton.

Thus according to a further feature of the invention there is provided a pharmaceutical or veterinary fungicidal composition which comprises a compound of formula I as defined above, or a salt, metal complex, ether or ester thereof, together with a pharmaceutically or veterinary acceptable diluent or carrier.

The following Examples illustrate the invention; the temperatures are given in degrees Centigrade (°C).

EXAMPLE 1

This Example illustrates the preparation of :

1-(1,2,4-Triazol-1-yl)-2-phenyl-2-(2-thienyl)ethan-2-ol (Compound no.1 of Table I)

A solution of dimethyl oxosulphonium methylide was prepared under nitrogen from sodium hydride (0.03 mol) and powdered trimethyl oxosulphonium iodide (0.03 mol) in dry dimethylsulphoxide (DMSO; 30 ml). A solution of phenyl (2-thienyl) ketone (0.025 mol) in DMSO (10 ml) was added drop wise at room temperature. The solution was then heated at 50° for 1.5 hours, cooled to room temperature and poured into water. The solution was extracted with diethyl ether (100 ml), washed with water (3 x 100 ml), and dried over anhydrous sodium sulphate. Removal of the solvent gave 1-phenyl-1-(2-thienyl) ethylene oxide (90%) as a colourless liquid.

1,2,4-Triazole (0.04 mol) was added portionwise to sodium hydride (0.04 mol) in DMF (40 ml) and the solution stirred at room temperature until effervescence ceased. 1-phenyl-1-(2-thienyl) ethylene oxide (0.02 mol) in DMF (10 ml) was added dropwise and the solution stirred at 80° for 4 hours. The solution was poured into water and triturated with petroleum ether to give a white crystalline solid which was filtered off and dried. Recrystallisation from petroleum ether (60-80°)/ methylene chloride gave the title compound (70%) as a white crystalline solid.

The remaining compounds of Table I above were prepared similarly using the appropriate starting materials.

EXAMPLE 2

This Example illustrates the preparation of :

<u>1-(1,2,4-Triazol-1-yl)-2-(2,4-dichlorophenyl)-2-(5-chlorothien-2-yl) ethan-2-ol (Compound No 11a of Table I)</u>

2, 4-Dichlorophenyl-5-chlorothien-2-yl ketone (14.6g), trimethylsulphoxonium iodide (12.1g), 1,2,4-triazole (3.8g) and potassium hydroxide pellets (6.2g) were refluxed in tertiary butanol (100 mls) for 3 hours. The reaction mixture was then cooled, poured into water (800 mls), extracted with methylene dichloride (2 x 800 mls), and the extractions combined and dried over anhydrous sodium sulphate. The solvent was then removed under vacuum and the resultant oil taken up in carbon tetrachloride. The insoluble material was filtered off and hexane added to the filtrate to yield a further solid which on recrystallisation from hexene/carbon tetrachloride gave the title compound m.p. 139-140°C.

EXAMPLE 3

This Example illustrates the preparation of :

<u>1-(1,2,4-Triazol-4-yl)-2-(2,4-dichlorophenyl)-2-(5-chlorothien-2-yl) ethan-2-ol Compound No 11b of Table I)</u>

The carbon tetrachloride-insoluble material from the above preparation was applied to 20 x 20 cms x 2 mm PLC plates (silica gel 60 $F_{254}$) in methanol, run in ethyl acetate and the required band, situated close to the base line, was extracted with methanol. The solid obtained was, on

BAD ORIGINAL

solvent removal, recrystallised from aqueous methanol to yield the title compound m.p. 247-8°C.

EXAMPLE 4

This Example illustrates a two-stage process for preparing :

1-(1,2,4-Triazol-1-yl)-2-(2,4-difluorophenyl)-2-(2-thienyl) ethan-2-ol (Compound No 60A of Table I)

Stage 1

A solution of dimethyl sulphoxonium methylide was prepared under nitrogen from 80% sodium hydride (0.6g) and powdered trimethyl sulphoxonium iodide (4.4.g) in dry dimethylsulphoxide (DMSO 30 mls). A solution of 2,4-difluorophenyl-2-thienyl ketone in DMSO (20 mls) was added dropwise at room temperature. The solution was then heated at 50°C for 1 hour, cooled and poured into water, extracted with ether (3 x 100 mls), washed with brine, and then with water before being dried over sodium sulphate. Removal of the solvent gave 1-(2,4-difluorophenyl)-1-(2-thienyl)-ethylene oxide as a dark brown oil.

Stage 2

1,2,4-Triazole (1.9g) was added portionwise to 80% sodium hydride (0.8g) in DMF (20 mls) and the solution stirred at room temperature until effervescence ceased. Oil (3.3g) prepared by Stage 1 was added dropwise in DMF (10 mls) and the solution stirred at 80°C for 2 hours. It was then cooled, poured into water and extracted with methylene dichloride and dried over sodium sulphate. Removal of the solvent gave an oil which solidified on trituration with ether. This was recrystallised from ether to yield the title compound m.p. 101-3°C.

EXAMPLE 5

An emulsifiable concentrate was made up ... ingredients, and stirring the mixture until all the constituents were dissolved.

| | |
|---|---|
| Compound No 1A of Table I | 10% |
| Ethylene dichloride | 40% |
| Calcium dodecylbenzenesulphate | 5% |
| "Lubrol" L | 10% |
| "Aromasol" H | 35% |

EXAMPLE 6

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound No 2A of Table 1 | 50% |
| "Dispersol" T | 25% |
| "Lubrol" APN5 | 1.5% |
| Sodium acetate | 23.5% |

EXAMPLE 7

The ingredients were all ground together to produce a powder formulation readily dispersible in liquids.

| | |
|---|---|
| Compound No 3A of Table I | 45% |
| "Dispersol" T | 5% |

BAD ORIGINAL

| | |
|---|---|
| "Lissapol" NX | 0.5% |
| "Cellofas" B600 | 2% |
| Sodium acetate | 47.5% |

EXAMPLE 8

The active ingredient was dissolved in a solvent and the resultant liquid was sprayed on to the granules of China clay. The solvent was then allowed to evaporate to produce a granular composition.

| | |
|---|---|
| Compound No 6A of Table I | 5% |
| China clay granules | 95% |

EXAMPLE 9

A composition suitable for use as a seed dressing was prepared by mixing the three ingredients.

| | |
|---|---|
| Compound No 8A of Table I | 50% |
| Mineral oil | 2% |
| China clay | 48% |

EXAMPLE 10

A dusting powder was prepared by mixing the active ingredient with talc.

| | |
|---|---|
| Compound No 9A of Table I | 5% |
| Talc | 95% |

EXAMPLE 11

A Col formulation was prepared by ball-milling the constituents set out below and then forming an aqueous suspension of the ground mixture with water.

| | |
|---|---|
| Compound No 10A of Table I | 40% |
| "Dispersol" T | 10% |
| "Lubrol" APN5 | 1% |
| Water | |

EXAMPLE 12

A dispersible powder formulation was made by mixing together the ingredients set out below and then grinding the mixture until all were thoroughly mixed.

| | |
|---|---|
| Compound No 11A of Table I | 25% |
| "Aerosol" OT/B | 2% |
| "Dispersol" A.C. | 5% |
| China clay | 28% |
| Silica | 40% |

EXAMPLE 13

This Example illustrates the preparation of a dispersible powder formulation. The ingredients were mixed and the mixture then ground in a comminution mill.

| | |
|---|---|
| Compound No 17A of Table I | 25% |
| "Perminal" BX | 1% |
| "Dispersol" T | 5% |
| Polyvinylpyrrolidone | 10% |
| Silica | 25% |
| China clay | 34% |

EXAMPLE 14

The ingredients set out below were formulated into a dispersible powder by mixing then grinding the ingredients.

| | |
|---|---|
| Compound No 18A of Table I | 25% |
| "Aerosol" OT/B | 2% |
| "Dispersol" A | 5% |
| China clay | 68% |

In Examples 5 to 14 the proportions of the ingredients given are by weight.

There now follows an explanation of the compositions or substances represented by the various Trade Marks and Trade Names mentioned above.

| | |
|---|---|
| LUBROL L : | a condensate of nonyl phenol 1 mole) with ethylene oxide (13 moles) |
| AROMASOL H : | a solvent mixture of alkylbenzenes |
| DISPERSOL T & AC : | a mixture of sodium sulphate and a condensate of formaldehyde with sodium naphthalene sulphonate |
| LUBROL APN5 : | a condensate of nonyl phenol (1 mole) with naphthalene oxide (5.5 moles) |
| CELLOFAS B600 : | a sodium carboxymethyl cellulose thickener |
| LISSAPOL NX : | a condensate of nonyl phenol (1 mole) with ethylene oxide (8 moles) |

AEROSOL OT/B : dioctyl sodium sulphosuccinate

PERMINAL BX : a sodium alkyl naphthalene sulphonate

EXAMPLE 15

A mixture of 5, 10, 25, 50, 100 or 250 parts of compound no.11A of Table I with 70 parts of calcium carbonate and 200 parts of a 10% maize starch past is dried and then passed through a 16 mesh screen. 5 parts of magnesium stearate are added and the granules are compressed to give a range of tablets suitable for oral administration for therapeutic purposes.

This active ingredient may be replaced by a therapeutically equivalent amount of any other triazole derivatives as hereinbefore defined.

EXAMPLE 16

A mixture of 2, 5, 10, 25, 50 or 100 parts of compound no.11A of Table I, 500 parts of lactose and 100 parts of maize starch is treated with sufficient 10% maize starch paste to give a granular mass. Each mixture is passed through a 16-mesh screen, dried, mixed with 8 parts of magnesium stearate and compressed into tablets, thus giving a range of tablets suitable for oral administration for therapeutic purposes.

The active ingredient may be replaced by a therapeutically equivalent amount of any other triazole derivatives as hereinbefore defined.

BAD ORIGINAL

EXAMPLE 17

A mixture of 10 parts of compound no.11A of Table I and 190 parts of wheat germ oil is filled into soft gelatin capsules, to give capsules suitable for oral administration for therapeutic purposes.

The active ingredient may be replaced by a therapeutically equivalent amount of any other triazole derivative as hereinbefore defined.

EXAMPLE 18

A solution of 10 parts of compound no.11A of Table I in 83 parts of water, 250 parts of glycerol and 125 parts of ethyl alcohol is mixed with a solution of 300 parts of sucrose in 150 parts of water. A suitable flavouring agent and colouring matter are then added to produce a syrup suitable for oral administration for therapeutic purposes.

The active ingredient may be replaced by a therapeutically equivalent amount of any other triazole derivative as hereinbefore defined.

EXAMPLE 19

A mixture of 3 parts of gum acacia and 1.5 parts of gum tragacanth is added to a mixture of 1 part of compound no. 11A of Table I and 33.7 parts of liquid paraffin. To the thoroughly triturated mixture is added slowly with stirring a solution of 0.1 part of cetyl alcohol-polyoxyethylene condensate, 40 parts of sucrose, 0.03 part of propyl p-hydroxybenzoate, 0.3 part of methyl p-hydroxybenzoate, a suitable flavouring agent and 0.002 part of

edible dyestuff in 110 parts of water. Th... then homogenized in conventional manner k... to produce an emulsion suitable for oral a... for therapeutic purposes.

The active ingredient may be replaced ... therapeutically equivalent amount of any other triazole derivative as hereinbefore defined.

EXAMPLE 20

A mixture of 0.5 part of finely divided compound no 11A of Table 1 propionamide in 3 parts of propylene glycol and 2 parts of ethylene glycol monoether was added to a stirred mixture of 4 parts of lanolin and 90.5 parts of molten soft white paraffin. The resulting mixture was allowed to cool to room temperature with rapid stirring, to give a uniform ointment containing 0.5% by weight of active ingredient suitable for topical administration for therapeutic purposes.

The active ingredient may be replaced by another triazole derivative as hereinbefore defined to give similar ointments.

EXAMPLE 21

A solution was prepared of 1 part of compound no.11A of Table 1 in 20 parts of ethanol and 27 parts of diethylene glycol monoethyl ester, then 50 parts of purified water was added, followed by 2 parts of a carboxypolymethylene gelling agent ("Carbapol 940" - Trade Mark) to give a finely dispersed gel suitable for topical administration for therapeutic purposes.

The active ingredient may be replaced by any other triazole or imidazole compound, or derivative, as hereinbefore described.

BAD ORIGINAL

EXAMPLE 22

The compounds were tested against a variety of foliar fungal diseases of plants. The technique employed was as follows.

The plants were grown in John Innes Potting Compost (No 1 or 2) in 4 cm diameter minipots. A layer of fine sand was placed at the bottom of the pots containing the dicotyledonous plants to facilitate uptake of test compound by the roots. The test compounds were formulated either by bead milling with aqueous Dispersol T or as a solution in acetone or acetone/ethanol which was diluted to the required concentration immediately before use. For the foliage diseases, suspensions (100 ppm active ingredient) were sprayed on to the soil. Exceptions to this were the tests on Botrytis cinerea and Venturia inaequalis. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the diseases. An exception was the test on Erysiphe graminis in which the plants were inoculated 24 hours before treatment. After inoculation, the plants were put into an appropriate environment to allow infection to take place and then incubated until the disease was ready for assessment. The period between inoculation and assessment varied from four to fourteen days according to the disease and environment. The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in Table II.

TABLE II

| COMPOUND NUMBER | PUCCINIA RECONDITA (WHEAT) | ERYSIPHE GRAMINIS (BARLEY) | PIRICULARIA ORYZAE (RICE) | BOTRYTIS CINEREA (GRAPE) | CERCOSPORA ARACHIDICOLA (PEANUT) | VENTURIA INEQUALIS (APPLE) |
|---|---|---|---|---|---|---|
| 1A | 3 | 4 | - | 0 | 0 | 0 |
| 3A | 4 | 4 | - | 4 | 3 | 4 |
| 6A | 4 | 4 | - | 4 | 3 | 4 |
| 8A | - | - | - | - | - | - |
| 9A | - | - | - | - | - | - |
| 10A | 4 | 4 | - | 0 | 3 | 4 |

TABLE II CONTINUED

| COMPOUND NUMBER | PUCCINIA RECONDITA (WHEAT) | ERYSIPHE GRAMINIS (BARLEY) | PIRICULARIA ORYZAE (RICE) | BOTRYTIS CINEREA (GRAPE) | CERCOSPORA ARACHIDICOLA (PEANUT) | VENTURIA INEQUALIS (APPLE) |
|---|---|---|---|---|---|---|
| 11A | 4 | 4 | – | – | 3 | 4 |
| 18A | 4 | 4 | – | 0 | 4 | 4 |
| 19A | – | – | – | – | – | – |
| 33A | 4 | 4 | – | 0 | 4 | 4 |
| 49A | 0 | 4 | – | 0 | 0 | 0 |
| [illegible] | 4 | 2 | – | 1 | 4 | 3 |

TABLE II CONTINUED

| COMPOUND NUMBER | PUCCINIA RECONDITA (WHEAT) | ERYSIPHE GRAMINIS (BARLEY) | PIRICULARIA ORYZAE (RICE) | BOTRYTIS CINEREA (GRAPE) | CERCOSPORA ARACHIDICOLA (PEANUT) | VENTURIA INEQUALIS (APPLE) |
|---|---|---|---|---|---|---|
| 57A | 4 | 4 | - | 4 | 4 | 4 |
| 1B | 4 | 4 | - | 0 | 0 | 0 |
| 2B | 3 | 4 | - | - | 0 | 0 |
| 3B | 4 | 4 | - | 0 | 4 | 3 |
| 9B | - | - | - | - | - | - |
| 10B | 0 | 4 | - | 0 | 0 | 0 |

TABLE II CONTINUED

| COMPOUND NUMBER | PUCCINIA RECONDITA (WHEAT) | ERYSIPHE GRAMINIS (BARLEY) | PIRICULARIA ORYZAE (RICE) | BOTRYTIS CINEREA (GRAPE) | CERCOSPORA ARACHIDICOLA (PEANUT) | VENTURIA INEQUALIS (APPLE) |
|---|---|---|---|---|---|---|
| 11B | 4 | 4 | - | 0 | 1 | 1 |
| 21B | 3 | 4 | - | - | 0 | 3 |
| 22B | 4 | 4 | - | - | 4 | 4 |
| 33B | 0 | 4 | - | 0 | 3 | 0 |

"-" means not tested

HGHA/aji SPEC147 & SPEC 147A

1. A racemic compound having the general formula (I) :

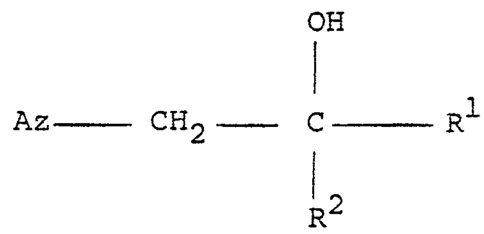

and stereoisomers thereof, wherein $R^1$ is alkyl, cycloalkyl (e.g. cyclopropyl, cyclopentyl or cyclohexyl) or optionally substituted phenyl and $R^2$ is a heterocyclic group; and Az is a 1, 2, 4-triazolyl radical; and acid addition salts, metal complexes, ethers and esters thereof.

2. A compound according to claim 1 wherein Az is a 1, 2, 4-triazol-1-yl radical or a 1, 2, 4-triazol-4-yl radical.

3. A triazole compound according to claim 1 or claim 2 wherein $R^1$ is an alkyl group which is a straight or branched chain alkyl group having from 1 to 6, especially 1 to 4, carbon atoms.

4. A triazole compound according to claim 1 or claim 2 wherein $R^1$ is phenyl or phenyl substituted with halogen, $C_{1-5}$ alkyl, $C_{1-4}$ alkoxy, halo-alkoxy, trifluoromethyl, nitro, phenyl or phenoxy.

5. A triazole compound according to any of claims 1, 2 and 4 wherein $R^1$ is phenyl, 2-, 3-, or 4-chlorophenyl, 2,4- or 2,6-dichlorophenyl, 2-, 3-, or 4-fluorophenyl, 2,4- or 2,6-difluorophenyl, 2-, 3-, or 4-bromophenyl, 2-chloro-4-fluorophenyl, 2-chloro-6-fluorophenyl, 2-, 3-, or 4-methoxyphenyl, 2-, 3-, or 4-nitrophenyl, 2-, 3-, or 4-methylphenyl, 2-, 3-, or 4-<u>t</u>-butylphenyl, 2-, 3-, or 4-trifluoromethylphenyl, 2-, 3-, or 4-phenoxyphenyl, and 2-, 3-, or 4-phenylphenyl (2-, 3-, or 4-biphenylyl).

6. A triazole compound according to any of claims 1 to 5 wherein $R^2$ is an unsubstituted, or halo- or alkyl-substituted, thiophene, furan, pyridine, pyrimidine or pyrrole ring.

7. A compound having the formula :

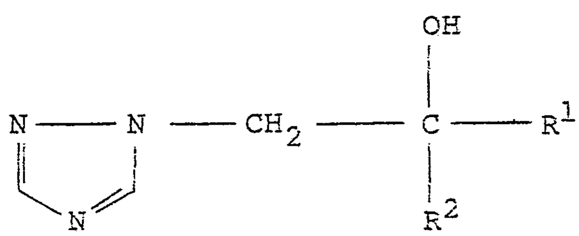

wherein $R^1$ is phenyl, 2- or 4-, chloro- or fluoro-, phenyl, or 2, 4-dichlorophenyl, or 2, 4-difluorophenyl, and $R^2$ is 2-thienyl, 2-[5-chlorothienyl], 2-furyl, 2-(5-butylfuryl), 2-, 3- or 4-pyridyl or 2-thienyl.

8. A compound according to claim 4 wherein $R^1$ is phenyl or halophenyl and $R^2$ is 2-[5-chlorothienyl] or 2-thienyl.

9. The compound :

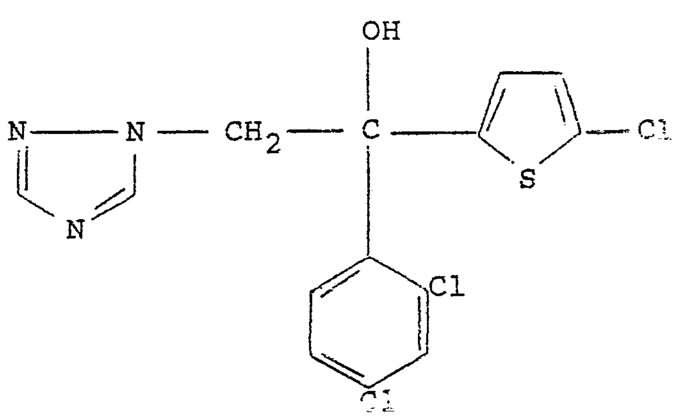

10. A process for preparing a compound as defined in any of claims 1 to 9 which comprises reacting a compound of general formula (II) or (III) :

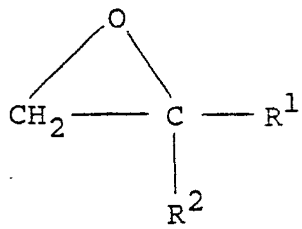

(II)

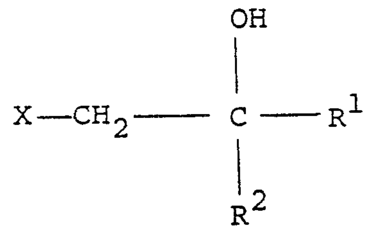

(III)

in which $R^1$ and $R^2$ are as defined in any of the preceeding claims and X is a halogen atom (preferably a chlorine or bromine atom), with 1, 2, 4-triazole, either in the presence of an acid-binding agent or in the form of one of its alkali metal salts, in a solvent.

11. A process according to claim 10 wherein the compounds of general formula (II) and (III) are prepared by reacting a compound of general formula (IVa) or (IVb) :

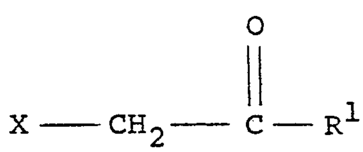

(IVa)

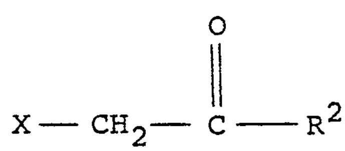

(IVb)

wherein $R^1$, $R^2$ and X are as defined above with, respectively, a Grignard compound of general formula (Va) or (Vb) :

$Y — Mg — R^2$

(Va)

$Y — Mg — R^1$

(Vb)

wherein $R^1$ and $R^2$ are as defined above and Y is a halogen (preferably chlorine, bromine or iodine) in a convenient solvent such as diethyl ether or tetrahydrofuran.

12. A process according to claim 10 wherein the compounds of general formula (II) wherein $R^1$ is unsubstituted or substituted phenyl and $R^2$ is a heterocyclic group are prepared by reacting the appropriate ketone of general formula (VI)

$$R^1 —— CO —— R^2$$

formula (VI)

wherein $R^1$ and $R^2$ are as defined with dimethyl oxosulphonium methylide or dimethyl sulphonium methylide.

13. A process according to claim 12 wherein the ketone of general formula (VI) is prepared, using the Friedel-Crafts reaction, or by methods set out in the literature.

14. As novel intermediates epoxides and halohydrins as defined in claim 10 and ketones as defined in claim 12.

15. A plant fungicidal composition comprising, as an active ingredient, a compound according to any of claims 1 to 9, or a salt, metal complex, ether or ester thereof; and a carrier or diluent therefor.

16. A method of combating fungal diseases in a plant, or for regulating plant growth, which method comprises applying to the plant, to seed of the plant, or to the locus of the plant or seed, a compound, or a salt, metal complex, ether or ester therefor, as defined in any of claims 1 to 9, or a composition as defined in claim 15.

17. A pharmaceutical or veterinary composition which comprises a compound according to any of claims 1 to 9, or a salt, metal complex, ether or ester thereof, together with a pharmaceutically or veterinary acceptable diluent or carrier.

18. A method of treatment of candidiasis or human dermatophyte infections which comprises administering to a human or animal an effective amount of a compound as claimed in any of claims 1 to 9 or a composition as claimed in claim 16.